# EUROPEAN PATENT APPLICATION

(11) **EP 4 641 173 A1**
(43) Date of publication of application: **29.10.2025**
(21) Application number: 25382395.9
(22) Date of filing: 16.04.2025
(51) Int. Cl.: G01N 21/64, G01N 33/03

(54) **METHOD TO DETECT IF A SAMPLE OF VIRGIN OLIVE OIL OR EXTRA VIRGIN OLIVE OIL IS ADULTERATED**

(30) Priority: 25.04.2024 ES 202430325
(71) Applicant: Universidad Pública De Navarra, 31006 Pamplona (ES)
(72) Inventor: VITORIA, Ignacio, 31180 Zizur Mayor (ES); RUIZ ZAMARREÑO, Carlos, 31006 PAMPLONA (ES); GRACIA MOISES, Ander, 31180 ZIZUR MAYOR (ES)
(74) Representative: Galbaian S.Coop.

(57) **Abstract**

Method for detecting if a sample of virgin olive oil VOO or extra virgin olive oil EVOO is adulterated, wherein the sample is illuminated at least once with an ultraviolet light having a given wavelength during a given time interval, and the spectrum of the light radiated by the sample is obtained at a plurality of instants of said time interval. It is determined whether or not the sample is adulterated based on the evaluation of the spectra obtained during said instants. Device for performing the method.

## Description

### TECHNICAL FIELD

The present invention relates to a method for detecting if a sample of virgin olive oil VOO or extra virgin olive oil EVOO is adulterated, and to a device for detecting if a sample of virgin olive oil VOO or extra virgin olive oil EVOO is adulterated or not.

### PRIOR ART

It is known to analyse the quality or adulteration of an oil sample by performing a chemical analysis or chromatography analysis of the oil sample. However, this method is slow, as it can take several hours to obtain the results.

It is also known to analyse the quality of an oil sample by a faster method, in which the sample is illuminated with infrared, visible or ultraviolet light and the quality of the sample can be determined by analysing the spectrum of light radiated by the sample.

For example, the paper *"*A comparative study of mid-infrared, UV-Visible and fluorescence spectroscopy in combination with chemometrics for the detection of adulteration of fresh olive oils with old olive oils", Food Control 105 (2019), 209-218, discusses the use of infrared light, visible light and ultraviolet light to analyse different olive oil samples.

On the other hand, EP2104854A1 relates to a method for determining quality in which oil is illuminated with an ultraviolet light with a first wavelength, for example, of about 470 nm, and the level of fluorescence of the oil is measured at a second wavelength, for example, of about 520 nm. Comparing the measured fluorescence level with a predetermined threshold level it is determined whether the quality of the oil is acceptable or not. This patent document also refers to a portable device for determining oil quality.

### DISCLOSURE OF THE INVENTION

The object of the invention is to provide a method for detecting if a sample of virgin olive oil VOO or extra virgin olive oil EVOO is adulterated, and a device for detecting if a sample of virgin olive oil VOO or extra virgin olive oil EVOO is adulterated or not, as defined in the claims.

A first aspect of the invention relates to a method for detecting if a sample of virgin olive oil VOO or extra virgin olive oil EVOO is adulterated.

In the method the sample is illuminated at least once with an ultraviolet light having a given wavelength for a given time interval, and the spectrum of the light radiated by the sample at a plurality of instants of said time interval is obtained. It is determined whether or not the sample is adulterated based on the evaluation of the spectra obtained during said instants.

Illumination of the sample with ultraviolet light causes the sample to degrade, so that different compounds in the sample change their fluorescence, causing the emission spectrum to vary with time and to change shape. Obtaining the spectrum of the sample at a plurality of instants while it is being illuminated, and the subsequent evaluation of said plurality of spectra, makes it possible to be able to evaluate said degradation of the sample. By evaluating said degradation, it is possible to determine more precisely if the analysed sample is adulterated or not.

A second aspect of the invention relates to a device for detecting if a sample of virgin olive oil VOO or extra virgin olive oil EVOO is adulterated or not.

The device comprises a central support configured to receive the sample to be analysed, light emitting means configured to emit ultraviolet light towards the sample, a detection system configured to measure the spectral response of the light radiated by the sample when said sample is illuminated by the light emitting means, and a control system configured to control the emitting means and the detection system.

The device is configured to perform a method according to the first aspect of the invention.

These and other advantages and features of the invention will become apparent in view of the figures and the detailed description of the invention.

### DESCRIPTION OF THE DRAWINGS

Figure 1 shows an example of the spectra of light radiated by a sample at a plurality of instants during illumination of said sample with a first 365 nm ultraviolet light.
Figure 2 shows in detail the spectra of Figure 1 in the range 450-560 nm.
Figure 3 shows in detail the spectra of Figure 1 in the range 640-780 nm.
Figure 4 shows an example of the spectra of the light radiated by the sample of Figure 1 at a plurality of instants during illumination of said sample with a second 395 nm ultraviolet light.
Figure 5 shows in detail the spectra of Figure 1 in the range 450-560 nm.
Figure 6 shows in detail the spectra of Figure 1 in the range 640-780 nm.
Figure 7 shows schematically the main elements of the device according to an embodiment of the invention.

### DETAILED DISCLOSURE OF THE INVENTION

The method of the invention makes it possible to detect if a sample 7 of virgin olive oil VOO or extra virgin olive oil EVOO is adulterated. For this purpose, the sample 7 is illuminated at least once with an ultraviolet light having a given wavelength during a given time interval, and the spectrum of the light radiated by the sample 7 at a plurality of instants of said time interval is obtained. The determination of whether or not the sample 7 is adulterated is made on the basis of the evaluation of the spectra obtained during said instants.

Illumination of the sample 7 with ultraviolet light causes said sample 7 to degrade. Obtaining the spectrum of the sample 7 at a plurality of instants while illuminated, and the subsequent evaluation of said plurality of spectra allows to be able to evaluate said degradation of the sample 7. The evaluation of said degradation allows a good determination of whether the sample 7 is adulterated or not.

Preferably the sample 7 is illuminated a first time with a first ultraviolet light with a first given wavelength during a first given time interval and a second time with a second ultraviolet light with a second given wavelength during a second given time interval, the spectrum of the light radiated by the sample 7 is obtained at a plurality of instants of the first time interval and at a plurality of instants of the second time interval, and it is determined whether or not the sample 7 is adulterated based on the evaluation of the spectra obtained during said instants. It has been observed that illuminating the sample 7 sequentially with two ultraviolet lights of different wavelengths and subsequently evaluating the spectra obtained during such sequential illumination provides a better determination of whether or not the sample 7 is adulterated.

When the sample is illuminated with said two lights of different wavelengths, preferably the first wavelength is shorter than the second wavelength. More preferably the first wavelength is less than 380 nm, preferably 365 nm, and the second wavelength is between 380 nm and 450 nm, preferably 395 nm. It has been observed that illumination of the sample with a first and second wavelength in such ranges provides an optimal adulteration determination of the sample 7.

In other possible embodiments, the sample 7 is illuminated a plurality of times, each of said times being illuminated with a given wavelength during a given time interval, at least one of said wavelengths being different from the others, the spectrum of the light radiated by the sample 7 is obtained at a plurality of instants of each of said intervals, and it is determined whether or not the sample 7 is adulterated based on the evaluation of the spectra obtained during said instants. In such cases, preferably, the sample 7 is illuminated with wavelengths of at least two defined ranges, one of said ranges comprising wavelengths shorter than 380 nm and the other range comprising wavelengths between 380 nm and 450 nm

Preferably the spectrum of the light radiated by the sample 7 is obtained at least at four instants of each time interval. More preferably the spectrum of the light radiated by the sample 7 is obtained at ten instants.

Preferably, for the determination of whether the sample 7 is adulterated or not, the ranges 450-560 nm and 640-780 nm of the spectra obtained are analysed. In these analysed ranges, the virgin olive oil VOO and the extra virgin olive oil EVOO present different peaks and shapes that depend on the organic compounds that form them, and the analysis of them results in the possibility of identifying adulterations or characterising the quality parameters.

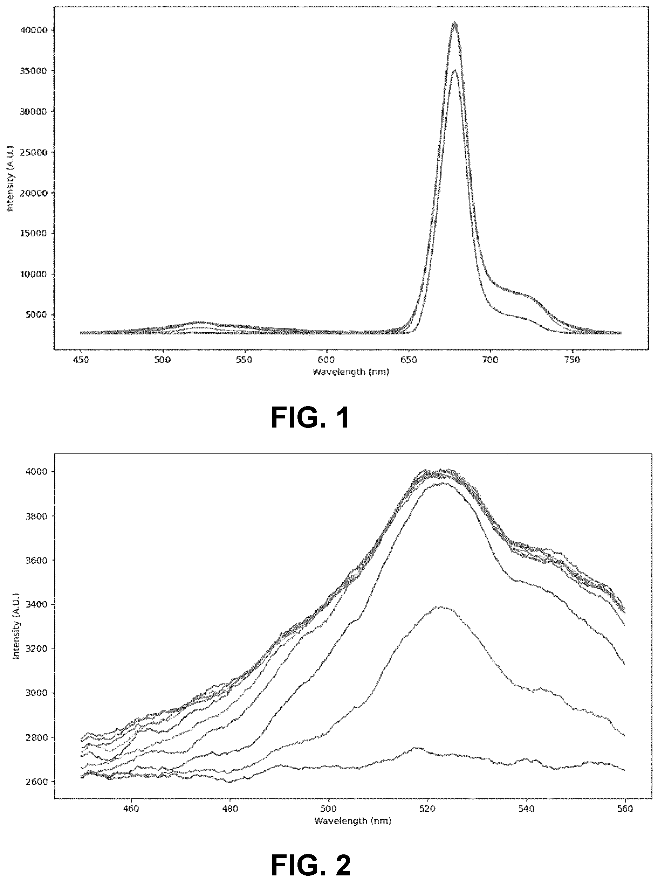

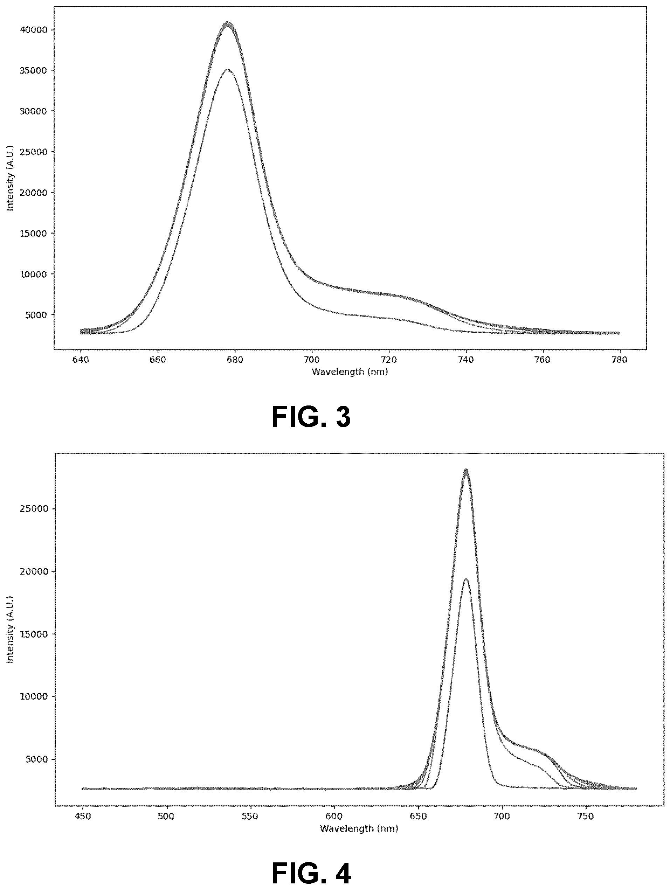

Figure 1 shows an example of spectra of the light radiated by a sample 7 at ten instants during the illumination of the sample with a first ultraviolet light of 365 nm. As can be seen in Figure 1, around the ranges 500-550 nm (see detail in Figure 2) and 650-750 nm (see detail in Figure 3) these spectra show an evolution in the form of a peak, being these ranges of the set of spectra the ones that contain more information about the sample 7 and specifically about the degradation of the same.

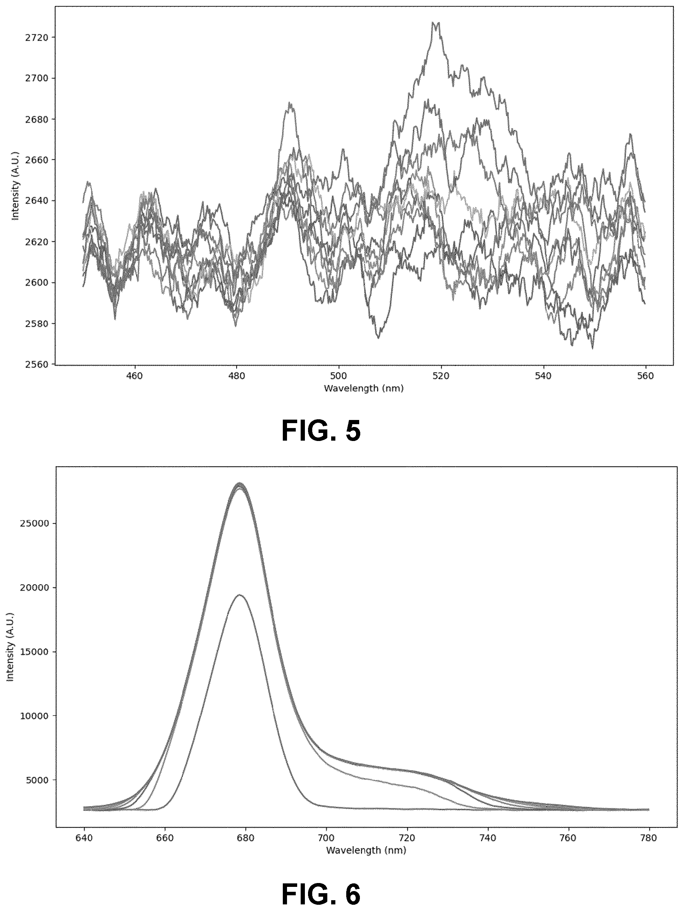

Figure 4 shows an example of spectra of the light radiated by sample 7 at ten instants during the illumination of the sample with a second ultraviolet light of 395 nm. As can be seen in Figure 4, around the range 450-560 nm (see detail in Figure 5) and especially around the range 640-780 nm (see detail in Figure 6) the spectra show an evolution in the form of a peak, being these ranges of the set of spectra the ones that contain more information about the sample 7 and specifically about the degradation of the same.

Preferably the instants of each time interval at which the spectra of the light radiated by the sample 7 are obtained are homogeneously distributed.

Preferably each time interval is between 0.2 and 1.4 seconds, more preferably about 1 second, since it has been observed that once this interval is exceeded the dynamics of the light radiated 7 by the sample tends to stabilize.

Preferably in the evaluation of the spectra obtained the concentration of stigmastadienes is determined, or if the amount of stigmastadienes comprising the sample 7 exceeds or does not exceed a predetermined value. VOO or EVOO oils should have a low concentration of stigmastadienes, and therefore it is a good parameter to analyse to know if the sample is adulterated or not. The presence of stigmastadienes in high concentrations is a clear indicator of adulteration, since these compounds usually appear in oils subjected to high temperatures or chemical processes that alter their original quality and properties. For example, in the case of the current International Olive Council IOC regulations, it is established that the maximum amount of stigmastadienes for an oil to be classified as EVOO must be equal to or less than 0.05 mg/kg. In the event that the amount of stigmastadienes exceeds the predetermined value, it would mean that the oil has been treated with a treatment additional to that normally used for its extraction or that it is mixed with another type of oil, for example with a lower quality olive oil or with a seed oil.

Preferably the set of spectra obtained is fed into a neural network trained to determine if the set of spectra fed into the network corresponds to an adulterated or unadulterated sample 7. For this purpose, preferably the neural network is trained with spectra obtained by illuminating adulterated and unadulterated samples, i.e. samples with an amount of stigmastadienes lower than the maximum amount established to be considered VOO or EVOO, and samples with an amount of stigmastadienes higher than the maximum amount established to be considered VOO or EVOO. In this way, the neural network learns to evaluate whether a set of spectra that are fed into the network refers to an adulterated oil sample or not. Preferably, the type of neural network employed is a convolutional network with dense layers, more preferably two convolutional layers and two dense layers, with a number of filters in the range of 32 up to 512, more preferably with a filter size of 64 and 128 respectively, for the purpose of extracting relevant features from the input data. Preferably there is also at least one *Dropout* layer with ratios of between 0.2 and 0.4, more preferably 0.25, before the output layer, with the aim of reducing the occurrence of overfitting. Preferably, to improve the stability and performance of the neural network training, use is made of batch normalization layers (BatchNormalization) and sigmoid, tanh, linear and relu functions are used as the activation function of each neuron, with preference given to the relu activation function.

Preferably the spectra obtained are normalized before feeding the set of spectra into the neural network. To this end, a transformation is applied to each spectrum to standardize the data, ensuring that the neural network operates on a consistent and optimized range of data. Preferably this transformation consists of fitting each spectrum according to the mean and standard deviation of its values, resulting in an electromagnetic spectrum with a mean of zero and a standard deviation of one, improving the efficiency and effectiveness of the neural network training.

A second aspect of the invention relates to a device 1 for detecting if a sample of virgin olive oil VOO or extra virgin olive oil EVOO is adulterated or not.

The device 1 of the invention comprises a central support 2 configured to receive the sample 7 to be analysed. Preferably the sample 7 is disposed in a cuvette, for example a cuvette having a capacity of 2.5 ml. The central support 2 of the device 1 may comprise a positioning system 20 to ensure that the sample 7 is always disposed in the same position with respect to the other elements of the device 1.

The device 1 also comprises light emitting means configured to emit ultraviolet light towards the sample 7. Preferably the light emitting means comprise at least one ultraviolet light source 30, 31, preferably an ultraviolet LED or an ultraviolet laser. Preferably said at least one ultraviolet light source 30, 31 is configured to emit light with a power of between 900 to 1300 mW and is arranged at a distance from the sample 7 of between 20 and 50 mm. Preferably, the light emitting means comprise a gate 32, 33 associated with each ultraviolet light source 30, 31, said gate 32, 33 being configured to allow or block the emission of light from the associated ultraviolet light source 30, 31 towards the sample 7. In this way, the gate 32, 33 ensures that the illumination of the sample 7 always meets the same criteria.

The device 1 also comprises a detection system configured to measure the spectral response of the light radiated by the sample 7 when said sample 7 is illuminated by the light emitting means. Preferably the detection system comprises a detector 40, more preferably a spectrometer. The detector 40 may be configured to directly receive the light radiated by the sample 7. In this case said detector is arranged perpendicularly with respect to the sample 7, preferably at a distance of 0.2 to 0.6 mm therefrom. Alternatively, the detection system may comprise in addition to the detector 40, a transmission means 41, preferably an optical fibre, said transmission means 41 being arranged perpendicularly to the light emitting means and configured to facilitate the efficient passage of the light radiated towards it by the sample 7 towards the detector 40 when said sample 7 is illuminated by the light emitting means.

The device 1 further comprises a control system 5 configured to control the light emitting means and the detection system. For example the control system may comprise a computer type control unit.

The device 1 is configured to execute a method as described in the first aspect of the invention.

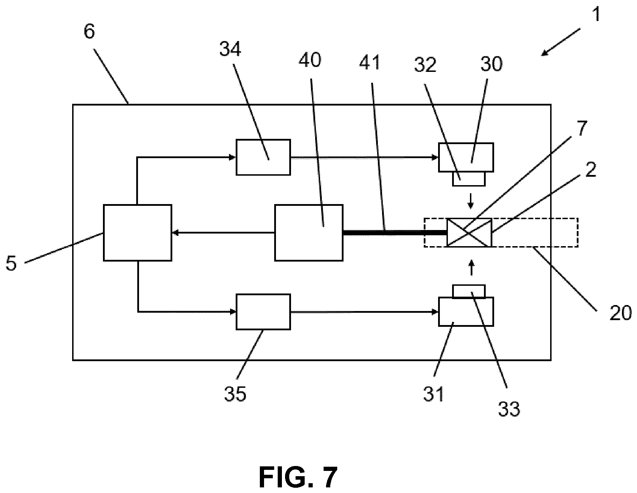

Figure 7 shows schematically the main elements of the device according to an embodiment of the invention.

The device 1 shown in Figure 7 comprises a central support 2 configured to receive a sample 7 to be analysed, said support 2 comprising a positioning system 20.

The device 1 shown in Figure 7 also comprises light emitting means comprising a first ultraviolet light source 30, and a second ultraviolet light source 31 facing the first ultraviolet light source 30, the light emitting means comprising a gate 32, 33 associated with each ultraviolet light source 30, 31. In addition, the light emitting means comprise a first driver 34 associated with the first ultraviolet light source 30 and a second driver 35 associated with the second ultraviolet light source 31.

The device 1 shown in Figure 7 also comprises a transmission means 41, preferably an optical fibre, arranged perpendicularly with respect to said ultraviolet light sources 30 and 31. The device 1 also comprises a detector 40, preferably a spectrometer. The transmission means 41 facilitates the efficient passage of the light radiated by the sample 7 towards the detector 40.

The device 1 shown in Figure 7 comprises a control system 5 configured to control the light emitting means and the detection system.

The device 1 shown in Figure 7 comprises a housing 6 forming a cavity in which the rest of the components of the device 1 are at least partially housed.

## Claims

1. Method for detecting if a sample of virgin olive oil VOO or extra virgin olive oil EVOO is adulterated, wherein the sample (7) is illuminated at least once with an ultraviolet light having a given wavelength during a given time interval, the spectrum of the light radiated by the sample (7) is obtained at a plurality of instants of said time interval, and it is determined whether or not the sample (7) is adulterated based on the evaluation of the spectra obtained during said instants.

2. Method according to claim 1, wherein the sample (7) is illuminated a first time with a first ultraviolet light with a first given wavelength during a first given time interval and a second time with a second ultraviolet light with a second given wavelength during a second given time interval, the spectrum of the light radiated by the sample (7) is obtained at a plurality of instants of the first time interval and at a plurality of instants of the second time interval, and it is determined whether or not the sample (7) is adulterated based on the evaluation of the spectra obtained during said instants.

3. Method according to claim 2, wherein the first wavelength is shorter than the second wavelength, the first wavelength being preferably less than 380 nm, more preferably 365 nm, and the second wavelength being preferably between 380 nm and 450 nm, more preferably 395 nm.

4. Method according to claim 1, wherein the sample (7) is illuminated a plurality of times, each of said times being illuminated with a given wavelength during a given time interval, at least one of said wavelengths being different from the others, the spectrum of the light radiated by the sample (7) is obtained at a plurality of instants of each of said intervals, and it is determined whether or not the sample (7) is adulterated based on the evaluation of the spectra obtained during said instants, wherein the sample (7) is preferably illuminated with wavelengths of at least two defined ranges, one of said ranges comprising wavelengths shorter than 380 nm and the other range comprising wavelengths between 380 nm and 450 nm.

5. Method according to any of the preceding claims, wherein the spectrum of the light radiated by the sample (7) is obtained in at least four instants of each time interval, preferably in ten instants.

6. Method according to any of the preceding claims, wherein the instants of each time interval are homogeneously distributed.

7. Method according to any of the preceding claims, wherein each time interval is between 0.2 and 1.4 seconds, preferably about 1 second.

8. Method according to any of the preceding claims, wherein the ranges 450-560 nm and 640-780 nm of the obtained spectra are analysed.

9. Method according to any of the preceding claims, wherein in the evaluation of the obtained spectra the concentration of stigmastadienes is determined, or whether or not the amount of stigmastadienes comprising the sample (7) exceeds a predetermined value.

10. Method according to any of the preceding claims, wherein the set of obtained spectra is fed into a neural network trained to determine if the set of spectra fed into the network corresponds to a sample (7) adulterated or not, wherein preferably the obtained spectra are normalized before feeding the set of spectra into the neural network.

11. Device for detecting if a sample of virgin olive oil VOO or extra virgin olive oil EVOO is adulterated or not, the device comprising
- a central support (2) configured to receive the sample (7) to be analysed,
- light emitting means configured to emit ultraviolet light towards the sample (7),
- a detection system configured to measure the spectral response of the light radiated by the sample (7) when said sample (7) is illuminated by the light emitting means, and
- a control system (5) configured to control the light emitting means and the detection system,
the device (1) being configured to execute a method according to any of the preceding claims.

12. Device according to claim 11, wherein the light emitting means comprise at least one ultraviolet light source (30, 31), preferably an ultraviolet LED or an ultraviolet laser, configured to emit light with a power of between 900 to 1300 mW, said at least one ultraviolet light source (30, 31) being arranged at a distance from the sample (7) of between 20 and 50 mm, the light emitting means preferably comprising a gate associated with the ultraviolet light source (30, 31), said gate being configured to allow or block the emission of light from the associated ultraviolet light source (30, 31) towards the sample (7).

13. Device according to claim 11 or 12, wherein the light emitting means comprise
- a first ultraviolet light source (30), and
- a second ultraviolet light source (31) facing the first ultraviolet light source (30).

14. Device according to any of claims 11 to 13, wherein the detection system comprises a detector (40), preferably a spectrometer, arranged perpendicularly with respect to the sample (7) and at a distance of 0.2 to 0.6 mm from the sample (7).

15. Device according to any of claims 11 to 13, wherein the detection system comprises a detector (40), preferably a spectrometer, and a transmission means (41), preferably an optical fibre, said transmission means (41) being arranged perpendicularly to the light emitting means and configured to facilitate efficient passage of light radiated by the sample (7) towards the detector (40) when said sample (7) is illuminated by the light emitting means.
